(19) **Europäisches Patentamt / European Patent Office / Office européen des brevets**

(11) **EP 1 173 481 B1**

(12) **FASCICULE DE BREVET EUROPEEN**

(45) Date de publication et mention
de la délivrance du brevet:
**26.11.2008 Bulletin 2008/48**

(21) Numéro de dépôt: **00922696.0**

(22) Date de dépôt: **20.04.2000**

(51) Int Cl.:
*C07K 14/52* (2006.01)    *C07K 14/525* (2006.01)
*C07K 14/54* (2006.01)    *C07K 14/56* (2006.01)
*C07K 14/57* (2006.01)    *C07K 1/06* (2006.01)

(86) Numéro de dépôt international:
**PCT/FR2000/001043**

(87) Numéro de publication internationale:
**WO 2000/064937 (02.11.2000 Gazette 2000/44)**

(54) **CYTOKINES INACTIVES POUR L'IMMUNISATION**

INAKTIVIERTE ZYTOKINE ZUR IMMUNISIERUNG

INACTIVATED CYTOKINES FOR IMMUNISATION

(84) Etats contractants désignés:
**AT BE CH CY DE DK ES FI FR GB GR IE IT LI LU MC NL PT SE**

(30) Priorité: **26.04.1999 FR 9905258**

(43) Date de publication de la demande:
**23.01.2002 Bulletin 2002/04**

(73) Titulaire: **NEOVACS**
**75116 Paris (FR)**

(72) Inventeurs:
• **ZAGURY, Daniel**
**F-75007 Paris (FR)**

• **ZAGURY, Jean-François**
**F-75003 Paris (FR)**

(74) Mandataire: **Catherine, Alain et al**
**Cabinet Harlé & Phélip**
**7 rue de Madrid**
**75008 Paris (FR)**

(56) Documents cités:
**WO-A- 94/21288        FR-A- 2 677 654**
**FR-A- 2 773 156        US-A- 4 414 147**

Remarques:
Le dossier contient des informations techniques présentées postérieurement au dépôt de la demande et ne figurant pas dans le présent fascicule.

**Description**

La présente invention concerne de nouvelles cytokines inactives, utilisables pour des immunisations chez l'homme.

PCT/FR92/00544 a divulgué qu'il était possible d'immuniser l'homme contre des cytokines après les avoir inactivées ou inactivé des fragments peptidiques natifs ou homologues par un traitement approprié conservant leur immunogénicité. Ce document décrit plus spécifiquement le traitement chimique de la cytokine ou fragment peptidique ou homologue à l'aide d'un aldéhyde ou de β propiolactone.

Ces composés se sont montrés très actifs. Ils ont notamment été utilisés dans le cadre d'essais chimiques sur patients infectés par le VIH, et ceux-ci ont montré un bénéfice chimique certain (voir A. Gringeri et al. J of AIDS 20 : 358-370 - 1999.

Mais ces composés, préparés par traitement au formaldéhyde, présentant parfois des difficultés de production, notamment sur le plan de la stabilité et de la reproductibilité.

Or la demanderesse a découvert avec étonnement que certains traitements chimiques permettaient d'obtenir des composés possédant la même immunogénicité, avec notamment une plus grande stabilité et reproductibilité de préparation.

C'est pourquoi la présente demande a pour objet un dérivé de cytokine ou de fragment de cytokine de formule

$$Cy\text{-}(X\text{-}R)_n \qquad (I)$$

dans laquelle Cy représente une cytokine ou un fragment de cytokine, -(X-R) représente une fonction libre d'un acide aminé constituant la cytokine ou le fragment de cytokine où X représente un groupement NH ou un atome de soufre, n représente un entier allant de 1 à 70, et R représente un groupement chimique conservant à ladite cytokine ou audit fragment des propriétés immunogènes suffisantes pour créer des anticorps neutralisant ou bloquant ladite cytokine native et en même temps faisant perdre au moins 90 % des propriétés biologiques de ladite cytokine native, **caractérisé en ce que** :

(i) R est un groupement carboxamide, N-alkylmaléimide, thionitrobenzoïque ou un radical formé par réaction d'une fonction thiol avec le 4-chloro-7-sulfobenzofurazane d'ammonium, le N-[iodoéthyl]-trifluoroacétamide ou le N-(6-[7-amino-4-méthylcoumarin-3-acétamido]hexyl)-3'-(2'-pyridyldithio) propionamide. -
La carboxamidation ajoute un radical carboxamide (traitement de la cytokine par l'iodoacétamide, par exemple), la maléimidation ajoute aux cystéines un radical N-alkylmaléimide, par exemple N-éthylmaléimide (traitement de la cytokine par la N-éthylmaléimide), la réaction d'Ellman ajoute un radical thionitrobenzoïque à la cystéine (traitement de la cytokine par de l'acide 5,5'-dithio-bis-[2-nitrobenzoïque] ou DTNB).

(ii) R est un radical formé par réaction d'une fonction amino avec l'éthylacétimidate, un anhydride, le 2-iminothiolane-HCl, le N-succinimidyl S-acétylthioacétate, le sulfosuccinimidyl-acétate, le sulfosuccinimidyl-4-O-[4,4'-diméthoxytrityl]butyrate, le succinimidyl 7-amino-4-méthylcoumarin-3-acétate ou le sulfosuccinimidyl 7-amino-4-méthylcoumarin-3-acétate ou le phénylglyoxal.

R a avantageusement un poids moléculaire compris entre 10 et 2000, de préférence entre 20 et 1500, notamment compris entre 30 et 1000, particulièrement compris entre 40 et 500.

R est de préférence un radical organique comme on l'a vu ci-dessus.

Le traitement de la cytokine par de l'éthylacétimidate produit l'amidination des fonctions amino. A titre d'anhydride utilisable on peut citer l'anhydride citraconique.

Comme on l'a vu, d'autres réactifs peuvent agir sur les fonctions amines comme par exemple le réactif de Traut (traitement par le 2-iminothiolane-HCl), par l'addition d'un SH protégé par le N-succinimidyl S-acétilthioacétate, ou le sulfosuccinimidyl-acétate ou le sulfosuccinimidyl-4-O-[4,4'-diméthoxytrityl]butyrate, ou encore le Succinimidyl 7-amino-4-méthylcoumarin-3-acétate ou le Sulfosuccinimidyl 7-amino-4-méthylcoumarin-3-acétate.

Ces inactivations chimiques sont obtenues par une modification chimique de certains résidus de la cytokine qui bloque la fonction biologique de la cytokine native. De préférence 50 % des groupes X ci-dessus sont modifiés, notamment 70 %, particulièrement la totalité. On peut modifier à la fois les groupes où X représente NH et ceux où X représente un atome de soufre, ou de préférence seulement un de ces deux groupes. "n" peut aller jusqu'à 10 par exemple dans le cas où X représente un atome de soufre et par exemple jusqu'à 70 par exemple dans le cas où X représente NH.

Le composé immunogène selon l'invention peut être constitué de la totalité ou d'un fragment de la cytokine et peut comporter, comme c'est bien connu de l'homme de l'art, une ou plusieurs modifications dans les acides aminés de cette protéine ou fragment telles que des délétions, substitutions, additions, ou fonctionnalisations telles qu'acylation d'acides aminés, dans la mesure où ces modifications restent dans le cadre précisé ci-dessus (absence de toxicité, caractères immunologiques). Par exemple, en général le remplacement d'un résidu leucine par un résidu isoleucine ne modifie pas de telles propriétés. Les modifications doivent généralement concerner moins de 30 % des acides aminés, de préférence moins de 20 % et tout particulièrement moins de 10 %.

Un fragment peut comporter de 8 à 110 acides aminés par exemple, de préférence de 12 à 60 acides aminés et notamment de 12 à 40 acides aminés. Un tel fragment peut comporter aussi du ou des côtés C ou N terminal de 1 à 5 acides aminés supplémentaires c'est-à-dire différents du segment d'origine.

De manière générale, en ce qui concerne les modifications, l'homologie ou la similitude entre l'immunogène modifié et la cytokine ou partie de cytokine native, ainsi que les dimensions du composé immunogène, de même que les modalités d'utilisation, ou de couplage du composé immunogène selon invention à une protéine immunogène telle que le toxoïde tétanique, on peut en particulier se référer à WO-A-86/06 414 ou à EP-A-0.220.273 ou encore à PCT/US. 86/00831.

La présente invention a encore pour objet un procédé de préparation d'une cytokine ou d'un fragment tel que défini ci-dessus, caractérisé en ce que l'on soumet ladite cytokine ou ledit fragment à l'action d'un réactif capable de greffer un groupement R sur ladite cytokine ou ledit fragment, pour obtenir le composé attendu que l'on isole.

Comme le montrent les exemples décrits dans la partie expérimentale ci-après et réalisés sur des cytokines d'origine humaine, les nouveaux produits obtenus par ces traitements, appelés " kinoïdes ", ont perdu leur activité biologique naturelle, et sont aptes à induire une réponse immunitaire chez le mammifère. De plus, les anticorps obtenus en utilisant ces kinoïdes comme immunogènes reconnaissent la protéine native non inactivée.

Les exemples cités se réfèrent aux cytokines, IFNγ, TNFα, IL1, IL4, IL6, IL10, IL13 mais ces réactions peuvent être appliquées à toutes les cytokines (les interleukines -IL1, IL2, IL3 etc..., les Transforming Growth Factors -TGF- α et β, les interférons α, β, γ, τ (tau), les Tumor Necrosis Factors -TNF α et β, les chimiokines etc...). La cytokine ou un fragment peptidique homologue peuvent être produits par synthèse chimique ou encore par génie génétique. Par fragment peptidique homologue, on entend une séquence peptidique qui a suffisamment de similitude avec la cytokine native pour induire des anticorps croisés qui neutraliseront l'activité biologique de la cytokine native. Par exemple, le fragment peptidique homologue peut être un mutant de la cytokine préparé par génie génétique. L'avantage de l'inactivation chimique est qu'elle permettra en plus de stabiliser la molécule pour obtenir des conformations et des préparations reproductibles.

Afin de maximiser la réponse immunitaire anti-cytokine, on pourra combiner l'utilisation de kinoïdes inactivés de manière différente et qui pourront être complémentaires sur le plan de la réaction immunitaire qu'ils induisent : par exemple un kinoïde préparé par une méthode ciblant les cystéines pourra être associé à un kinoïde préparé selon une méthode ciblant les résidus amines.

Les kinoïdes pourront être associés pour lutter contre des surproductions de cytokines, non seulement des kinoïdes de la même cytokine inactivés par des méthodes différentes, mais aussi des kinoïdes de cytokines différentes surproduites dans la même maladie : par exemple dans le cas du SIDA on pourra immuniser en utilisant des kinoïdes combinés d'IFNα et d'IFNγ, ou dans la polyarthrite rhumatoïde en utilisant des kinoïdes d'IL1 et de TNFα.

Les composés objet de la présente invention possèdent de très intéressantes propriétés pharmacologiques. Ils sont particulièrement intéressants pour l'immunisation active anti-cytokine dans les cas où des maladies sont associées à une surproduction de ces cytokines.

Ces propriétés sont illustrées ci-après dans la partie expérimentale. Elles justifient l'utilisation des composés de formule (I) ci-dessus décrits, à titre de médicament.

C'est pourquoi l'invention a aussi pour objet les composés répondant à la formule I

$$Cy\text{-}(X\text{-}R)_n \qquad (I)$$

dans laquelle Cy représente une cytokine ou un fragment de cytokine, -(X-R) représente une fonction libre d'un acide aminé constituant la cytokine ou le fragment de cytokine où X représente un groupement NH ou un atome de soufre, n représente un entier allant de 1 à 70, et R représente un groupement chimique conservant audit dérivé de cytokine ou audit fragment des propriétés immunogènes suffisantes pour créer des anticorps neutralisant ou bloquant ladite cytokine native et en même temps faisant perdre au moins 90 % des propriétés biologiques de ladite cytokine native, pour leur utilisation dans une méthode de traitement thérapeutique du corps humain ou animal, c'est-à-dire à titre de médicament.

Les médicaments selon la présente invention trouvent leur emploi dans des traitements tant curatifs que préventifs. De manière générale, ils peuvent être employés dans les pathologies associées à une surproduction, ou production indésirable de cytokine. Par exemple, le kinoïde de TNFα peut être utilisé en immunisation active dans la polyarthrite rhumatoïde où il a déjà été montré que le blocage de TNF par des anticorps (immunisation passive) ou des récepteurs solubles limite l'évolution de la maladie, ou encore dans les cancers quand il y a surproduction de TNFα et cachexie. Le kinoïde d'IL1 peut aussi être utilisé dans la polyarthrite rhumatoïde où il y a surproduction d'IL1. Le kinoïde d'IFNγ peut-être utilisé dans le SIDA où il y a surproduction d'IFNγ ; il peut être utilisé dans la sclérose en plaques où il a été montré que l'administration d'IFNγ entraîne une aggravation de la maladie, ou encore dans le diabète juvénile où il a été clairement montré sur modèle animal et aussi par des études in situ chez l'homme que la destruction des îlots de Langerhans est due à une surproduction d'IFNγ. Le kinoïde d'IFNα peut être utilisé dans le SIDA où sa surproduction a été clairement établie et où le bénéfice clinique de l'immunisation anti-IFNα a été démontré ou encore dans le lupus érythémateux dont il a été démontré qu'il pouvait être déclenché par injection d'IFNα. Le kinoïde d'IL6 peut être utilisé dans le myélome multiple ou la maladie de Castleman ou certains mélanomes où il a été montré que les cellules cancéreuses prolifèrent grâce à l'IL6. Les kinoïdes d'IL4 et d'IL13, isolés ou ensemble, peuvent être utilisés dans des cas de pathologies allergiques où il a été montré que ces cytokines sont surproduites. Tous ces exemples non limitatifs montrent l'éventail large d'applications des kinoïdes décrits dans ce brevet.

Comme pour toute immunisation active, le traitement peut n'utiliser qu'un fragment de cytokine inactivé par les méthodes mentionnées ci-dessus pour susciter la réaction immunitaire qui neutralisera l'activité biologique de la cytokine.

Les composés immunogènes selon l'invention peuvent être utilisés comme suit :

On administre à un patient un composé immunogène selon la présente invention, par exemple par voie sous-cutanée ou intramusculaire, en quantité suffisante pour être efficace sur le plan thérapeutique, à un sujet ayant besoin d'un tel traitement. La dose administrée peut aller par exemple de 20 à 1000 μg par voie intra musculaire sous forme d'émulsion e/h, une fois par mois pendant trois mois, puis périodiquement en fonction du taux des anticorps sériques induits, par exemple tous les 2-6 mois.

Une composition selon l'invention peut être administrée par n'importe quelle voie conventionnelle en usage dans le domaine des vaccins, en particulier par voie sous-cutanée, par voie intramusculaire, par voie intraveineuse ou par voie orale. On peut aussi citer la voie oro-muqueuse ou pernasale. L'administration peut avoir lieu en dose unique ou répétée une ou plusieurs fois après un certain intervalle de temps.

L'invention a aussi pour objet les compositions pharmaceutiques qui renferment au moins un composé précité, à titre de principe actif.

Dans ces compositions, le principe actif est avantageusement présent à des doses physiologiquement efficaces ; les compositions précitées renferment notamment une dose immunogène efficace d'au moins un principe actif ci-dessus. Le composé immunogène peut être conditionné seul ou mélangé à un excipient pharmaceutiquement acceptable tel qu'un adjuvant.

Ces compositions pharmaceutiques peuvent être, notamment liquides et se présenter sous les formes pharmaceutiques couramment utilisées en médecine humaine pour les vaccins, comme par exemple les préparations injectables notamment sous forme d'émulsion ; elles sont préparées selon les méthodes usuelles. Le ou les principes actifs peuvent y être incorporés à des excipients habituellement employés dans ces compositions pharmaceutiques, tels que les véhicules aqueux, le phosphate de calcium, l'alun....

La présente invention a encore pour objet un procédé de préparation d'une composition ci-dessus décrite, caractérisé en ce que l'on mélange, selon des méthodes connues en elles mêmes, le ou les principes actifs avec des excipients acceptables, notamment pharmaceutiquement acceptables.

L'invention a enfin pour objet l'utilisation d'une cytokine ou un fragment de cytokine de formule

$$Cy\text{-}(X\text{-}R)_n \qquad (I)$$

dans laquelle Cy représente une cytokine ou un fragment de cytokine, -(X-R) représente une fonction libre d'un acide aminé constituant la cytokine ou le fragment de cytokine où X représente un groupement NH ou un atome de soufre, n représente un entier allant de 1 à 70, et R représente un groupement chimique conservant à ladite cytokine ou audit fragment des propriétés immunogènes suffisantes pour créer des anticorps neutralisant ou bloquant ladite cytokine native et en même temps faisant perdre au moins 90 % des propriétés biologiques de ladite cytokine native, pour l'obtention d'un médicament destiné à lutter contre la surproduction de ces cytokines natives.

En outre l'invention a pour objet un kit comprenant une composition pharmaceutique vaccinale qui en plus du principe actif (par exemple cytokine ou fragment de cytokine) peut comprendre un adjuvant et/ou un autre immunogène à propriétés anti-surproduction de ces cytokines natives.

Les conditions préférentielles de mise en oeuvre des cytokines ou fragments de cytokines ci-dessus décrites s'appliquent également aux autres objets de l'invention visés ci-dessus.

Les exemples qui suivent illustrent la présente demande.

Exemple 1. Traitement de l'IFNγ par maléimidation

On a mis 10 mg d'IFNγ recombinant humain en solution à 0,5 mg par ml dans du tampon phosphate 0,1 M pH

**4**

7. On a ajouté à cette solution 1 mg de N-éthylmaléimide. Après 1 heure de réaction, on a dialysé la solution contre PBS pH 7,2 L'analyse du kinoïde par gel d'acrylamide montre la même bande à 17 KD comparée à l'IFNγ natif.

Exemple 2. Perte de l'activité biologique de l'IFNγ inactivé par maléimidation

On a ensuite testé le kinoïde IFNγ pour évaluer son activité biologique. Il y a plusieurs tests biologiques d'activité de l'IFNγ. Des tests d'induction d'apoptose, des tests d'induction d'antigènes de surface comme les MHC (complexe majeur d'histocompatibilité), mais aussi le test d'effet antiviral sur cellules MDBK.

Le test de mesure d'effet antiviral a pour but d'évaluer l'inhibition de la lyse de cellules MDBK en présence d'IFNγ. Ce test est très sensible et permet de mesurer des picogrammes d'IFNγ.

On fait pousser des cellules MDBK fraîchement trypsinées en milieu RPMI, sérum de veau foetal 5%, à la concentration de 30 000 cellules par puits. Après au moins 6 heures, on lave les cellules au RPMI, puis on ajoute 50 ml d'IFNγ dilué dans du RPMI en série de dilutions au 1/2. La plaque est alors incubée à 37°C 5% $CO_2$ pendant 18 à 24 heures.

Après 18 à 24 heures, le surnageant de chaque puits est enlevé, les cellules sont lavées, et on ajoute 100 ml contenant 100 DL50 (dose léthale 50%) de virus VSV dilué dans du RPMI. On laisse la plaque incuber ainsi pendant 18 à 36 heures. L'effet lytique du virus se mesure sous le microscope et permet de quantifier (échelle 1 à 4) à partir de quelle dilution la lyse s'opère.

Pour tester l'activité du kinoïde comparé à l'IFNγ natif, on part de solutions contenant 100 ng/ml de ces molécules. On obtient le résultat suivant :

|  | IFNγ natif | kinoïde IFNγ maléimidé |
|---|---|---|
| Effet antiviral positif | Jusqu'à dilution $2^{10}$ | Jusqu'à dilution $2^2$ |

Le kinoïde a donc perdu son activité antivirale et est inactif à un facteur supérieur à $2^8$ dans ces conditions, ce qui correspond à une inactivation supérieure à 99%.

Exemple 3. Immunogénicité de l'IFNγ maléimidé.

Des kinoïdes préparés selon l'exemple 1 ont été utilisés pour immuniser des souris. Le protocole d'immunisation est celui classiquement utilisé : on injecte à des souris, par voie intramusculaire (im), 100 ml d'émulsion (1 : 1) en adjuvant complet de Freund contenant 20 mg de produit au jour 0, avec rappel en adjuvant incomplet de Freund de 5 mg aux jours 21 et 35. Le sérum des souris est prélevé aux jours -2 et 40 et analysé par ELISA sur des plaques sensibilisées avec la protéine native (non traitée chimiquement) et avec du kinoïde. Les sérums ont été testés à une dilution au 1/500.

Les résultats, exprimés en densité optique, obtenus sur 3 souris immunisées (1 à 3) et 3 souris non immunisées (4 à 6), sont rapportés dans le tableau suivant :

|  |  | Protéine native | Kinoïde |
|---|---|---|---|
| Souris 1 | J-2 | 0,1 | 0,2 |
|  | J40 | 1,8 | 2,1 |
| Souris 2 | J-2 | 0,15 | 0,2 |
|  | J40 | 1,5 | 2,2 |
| Souris 3 | J-2 | 0,3 | 0,1 |
|  | J40 | 2 | 2,1 |
| Souris 4 | J-2 | 0,2 | 0,15 |
|  | J40 | 0,2 | 0,1 |
| Souris 5 | J-2 | 0,15 | 0,2 |
|  | J40 | 0,1 | 0,15 |
| Souris 6 | J-2 | 0,15 | 0,2 |
|  | J40 | 0,2 | 0,1 |

Ces résultats montrent que les souris immunisées par le kinoïde produisent des anticorps capables de reconnaître de la même façon la protéine native et le kinoïde. D'autre part, cela confirme l'innocuité de l'immunisation par les kinoïdes car les souris ont très bien toléré l'immunisation.

Exemple 4. Traitement du TNFα par carboxamidation.

3 mg de TNFα dissous dans 2 ml de tampon Tris 0,3 M, Guanidine 6 M EDTA 10 mM, DTT 1 mM ont été traités par 107 ml d'une solution d'iodacétamide 0,5 M sous une barrière d'azote pendant 1 h30 à 37°C. Après blocage de la réaction par addition de 2,5 ml de β-mercaptoéthanol, le mélange réactionnel a été dialysé successivement contre de l'urée 8 M, 4 M, 2 M et contre tampon phosphate (PBS).

Exemple 5. Perte de l'activité biologique du TNFα préparé par carboxamidation.

Il existe plusieurs essais mesurant l'activité cytotoxique du TNFα sur des lignées cellulaires que ce soit la lignée L929 de souris ou la lignée WEHI164. Le principe de l'essai sur lignée WEHI 164 est que le TNFα va induire une lyse cellulaire mesurée par crystal violet.

50 000 cellules sont déposées dans des puits de plaques 96 puits Costar, dans 100 ml de RPMI SVF 10%. On laisse reposer 4 heures à 37°C. A ces puits on ajoute alors en triple 100 ml de RPMI contenant des dilutions croissantes de TNFα natif ou inactivé. Les dilutions se font de 5 en 5. Après 24H à 37°C, on enlève le surnageant et on fixe les cellules dans chaque puits avec 200 ml de méthanol pendant 30 s. Après avoir enlevé le méthanol on ajoute on ajoute du crystal violet en solution à 1 % pendant 10 mn. Ensuite on lave les puits à l'eau distillée, puis on laisse sécher la plaque en la retournant sur du papier absorbant. Après séchage, on mesure le marquage à 620 nm dans un lecteur de plaques.

Le pourcentage de lyse se calcule par la formule :

$$\% = (DO_{max} - DO_{exp}) / (DO_{max} - DO_{min}) \times 100$$

où max correspond à l'absence de lyse (contrôle RPMI seul) et min correspond à la lyse maximum (HCL ou TNFα natif en excès).

Les résultats (% de lyse) obtenus pour le TNFα natif et le TNFα inactivé selon l'exemple 4 sont les suivants :

| Quantité/ puits : | TNFα natif | TNFα inactivé |
|---|---|---|
| 10 mg | 100% | 5% |
| 1 mg | 100% | 0% |
| 100 ng | 100% | 5% |
| 10 ng | 100% | 2% |
| 1 ng | 100% | 0% |
| 100 pg | 98% | 3% |
| 10 pg | 65% | 2% |
| 1 pg | 5% | 4% |

On voit que quelles que soit les doses de TNFα inactivé, il n'a plus d'effet lytique sur les cellules (bruit de fond), alors que le TNFα natif est actif jusqu'à une dilution d'un facteur $10^5$ par rapport à la dilution initiale.

Exemple 6. Immunogénicité du TNFα carboxamidé.

Des kinoïdes préparés selon l'exemple 4 ont été utilisés pour immuniser des souris. Le protocole d'immunisation est celui classiquement utilisé : on injecte à des souris, (en im), 100 ml d'émulsion (1 : 1) en adjuvant complet de Freund contenant 20 mg de produit au jour 0, avec rappel en adjuvant incomplet de Freund de 5 mg aux jours 21 et 35. Le sérum des souris est prélevé aux jours -2 et 40 et analysé par ELISA sur des plaques sensibilisées avec la protéine native (non traitée chimiquement) et avec du kinoïde. Les sérums ont été testés à une dilution au 1/500.

Les résultats, exprimés en densité optique, obtenus sur 3 souris immunisées (1 à 3) et 3 souris non immunisées (4 à 6), sont rapportés dans le tableau suivant :

|  |  | Protéine native | Kinoïde |
|---|---|---|---|
| Souris 1 | J-2 | 0,1 | 0,15 |
|  | J40 | 1,9 | 1,7 |
| Souris 2 | J-2 | 0,1 | 0,2 |
|  | J40 | 1,7 | 2 |
| Souris 3 | J-2 | 0,2 | 0,2 |
|  | J40 | 1,8 | 1,8 |
| Souris 4 | J-2 | 0,2 | 0,3 |
|  | J40 | 0,1 | 0,1 |
| Souris 5 | J-2 | 0,2 | 0,2 |
|  | J40 | 0,15 | 0,2 |
| Souris 6 | J-2 | 0,15 | 0,2 |
|  | J40 | 0,2 | 0,15 |

Ces résultats montrent que les souris immunisées par le kinoïde produisent des anticorps capables de reconnaître de la même façon la protéine native et le kinoïde. D'autre part, cela confirme l'innocuité de l'immunisation par les kinoïdes car les souris ont très bien toléré l'immunisation.

Exemple 7. Traitement de l'IL4 par réactif d'Ellman

1 mg d'IL4 en tampon phosphate 0,1 M, pH 8,0 a été traité par 4 mg de DTNB pendant 30 mn à température ambiante, à l'abri de la lumière. Le mélange a été ensuite dialysé contre PBS.

Exemple 8. Perte de l'activité biologique de l'IL4 traité par le réactif d'Ellman.

L'IL4 a de nombreux effets sur l'activation-différentiation des lymphocytes B avec l'induction d'expression de marqueurs de surface comme le MHC de classe II, CD40, CD23 qui peuvent être évalués par cytofluorométrie. Dans l'expérience présente, on mesure la prolifération de cellules mononucléées du sang périphériques (PBMCs) activées par phytohémagglutinine (PHA) et IL2, en présence de différentes concentrations d'IL4.
Les PBMCs sont purifiés sur gradient de Ficoll. 5 millions de cellules sont mises en flasque dans 5 ml de RPMI, SVF %, avec ajout de PHA 1/1000 et la culture est poursuivie 48 heures à 37° 5% $CO_2$. De l'IL2 est alors ajoutée à la concentration de 20 UI/ml, et après 48H, les cellules sont centrifugées, lavées et mises à concentration de 20 000 cellules par puits dans une plaque de culture de 96 puits en présence de RPMI, sérum de veau foetal (SVF) 5%, PHA 1/1000. A ces puits on ajoute différentes quantités d'IL4 dilué en RPMI. Les plaques sont alors incubées 40 heures, puis on leur ajoute de la thymidine tritiée, et après 6 heures, on mesure la prolifération cellulaire en évaluant l'incorporation de thymidine marquée dans un compteur β.
Les résultats de prolifération (cpm) obtenus en présence d'IL4 natif ou inactivé selon l'exemple 7 sont les suivants :

| Quantité par puits : | IL4 natif | IL4 inactivé |
|---|---|---|
| 100 ng | 49 000 | 400 |
| 10 ng | 25 000 | 300 |
| 1 ng | 6 000 | 400 |
| 0,1 ng | 1 000 | 300 |

On voit que l'inactivation est efficace même après une dilution d'un facteur 1000, ce qui montre une inactivation biologique de plus de 99,9%.

Exemple 9. Immunogénicité de l'IL4 inactivée par réactif d'Ellman.

Des kinoïdes préparés selon l'exemple 7 ont été utilisés pour immuniser des souris. Le protocole d'immunisation est celui classiquement utilisé : on injecte à des souris, (en im), 100 ml d'émulsion (1 : 1) en adjuvant complet de Freund contenant 20 mg de produit au jour 0, avec rappel en adjuvant incomplet de Freund de 5 mg aux jours 21 et 35. Le sérum-des souris est prélevé aux jours -2 et 40 et analysé par ELISA sur des plaques sensibilisées avec la protéine native (non traitée chimiquement) et avec du kinoïde. Les sérums ont été testés à une dilution au 1/500.

Les résultats, exprimés en densité optique, obtenus sur 3 souris immunisées (1 à 3) et 3 souris non immunisées (4 à 6), sont rapportés dans le tableau suivant :

|  |  | protéine native | kinoïde |
|---|---|---|---|
| Souris 1 | J-2 | 0,2 | 0,1 |
|  | J40 | 2 | 1,9 |
| Souris 2 | J-2 | 0,3 | 0,15 |
|  | J40 | 2,1 | 1,9 |
| Souris 3 | J-2 | 0,3 | 0,2 |
|  | J40 | 2,1 | 1,9 |
| Souris 4 | J-2 | 0,15 | 0,2 |
|  | J40 | 0,1 | 0,25 |
| Souris 5 | J-2 | 0,1 | 0,15 |
|  | J40 | 0,2 | 0,2 |
| Souris 6 | J-2 | 0,1 | 0,1 |
|  | J40 | 0,15 | 0,3 |

Ces résultats montrent que les souris immunisées par le kinoïde produisent des anticorps capables de reconnaître de la même façon la protéine native et le kinoïde. D'autre part, cela confirme l'innocuité de l'immunisation par les kinoïdes car les souris ont très bien toléré l'immunisation.

Exemple 10. Traitement de l'IL6 par amidination.

A 4 ml d'une solution d'IL6 à 1 mg/ml en tampon borate 0,1 M, pH 8,5 refroidie dans un bain de glace, on ajoute, sous agitation, 40 ml d'une solution d'éthylacétimidate à 12,5 mg/ml dans NaOH 5 N. Le mélange est agité pendant 30 à 60 mn, dans le bain de glace tout en maintenant le pH à 8,5 par addition de NaOH 0,1 N, après quoi, le mélange est dialysé contre PBS.

Exemple 11. Perte de l'activité biologique de l'IL6 traité par amidination.

Le test de mesure de l'activité de l'IL6 repose sur la mesure de la prolifération d'une lignée cellulaire dépendant d'IL6 pour sa croissance. Des cellules 7TD1 sont cultivées dans le milieu de culture suivant : milieu de Eagle modifié selon Dulbecco (DMEM) avec 10% de sérum de veau foetal, 1,5 mM Glutamine, 0,24 mM Asparagine, 0,55 mM Arginine, 50 mM $\beta$-Mercaptoéthanol, 0,1 mM hypoxanthine et 16 mM thymidine. Ces cellules sont normalement cultivées en présence d'IL6 native à la concentration de 200 U/ml soit de l'ordre de 1 ng/ml d'IL6.

On lave les cellules 2 fois dans du milieu DMEM seul et on les re-suspend dans le milieu de culture à la concentration de 20 000 cellules par ml. 100 ml de ces cellules sont ajoutées à des séries diluées d'IL6 (dans le même volume de 100 ml) ou d'échantillon à tester.

Après incubation 3-4 jours à 37°C et 8% CO2, le nombre de cellules vivantes est évalué par colorimétrie en mesurant le niveau d'hexosaminidase (Landegren et al., J Immunol Méthods, 67, 379, 1984) : pour cela, les microplaques sont centrifugées, les cellules sont lavées deux fois en PBS pour enlever le sérum, le substrat colorant est ajouté (1 volume de p-nitrophényl-N-acétyl-b-D-glucosaminide 7,5 mM, 0,1 M sodium citrate pH 5, 1 volume de Triton X100 à 0,5%) à 60 ml par puits. Après incubation 4 heures à 37°C, le révélateur est ajouté (0,1 M glycine-NaOH, pH 10,4). La couleur est lue sous absorbance 405 nm avec un contrôle blanc à 620 nm.

Une unité d'IL6 correspond à 50% de la prolifération maximale obtenue selon le calcul suivant :

$$\text{Prolif max 50} = \text{prolif contrôle} + 1/2 \,(\text{prolif max-prolif contrôle}).$$

Dans le cas des préparations de l'exemple 10, on a testé à chaque fois 5 ng de préparation dans 100 ml et réalisé les dilutions de 3 en 3. Les dilutions donnant une unité sont données dans le tableau suivant :

|  | Dilution pour obtenir 1 Unité | % d'inactivation |
|---|---|---|
| IL6 natif | $3^6$ | 0% |
| Kinoïde IL6 | 3 | 99,8 |

Ces résultats montrent la disparition de l'activité biologique IL6 à plus de 99,8% chez le kinoïde IL6 inactivé par amidination comparé à l'IL6 natif.

Exemple 12. Immunogénicité de l'IL6 inactivée par amidination.

Des kinoïdes préparés selon l'exemple 10 ont été utilisés pour immuniser des souris. Le protocole d'immunisation est celui classiquement utilisé : on injecte à des souris, (en im), 100 ml d'émulsion (1 : 1) en adjuvant complet de Freund contenant 20 mg de produit au jour 0, avec rappel en adjuvant incomplet de Freund de 5 mg aux jours 21 et 35. Le sérum des souris est prélevé aux jours -2 et 40 et analysé par ELISA sur des plaques sensibilisées avec la protéine native (non traitée chimiquement) et avec du kinoïde. Les sérums ont été testés à une dilution au 1/500.

Les résultats, exprimés en densité optique, obtenus sur 3 souris immunisées (1 à 3) et 3 souris non immunisées (4 à 6), sont rapportés dans le tableau suivant :

|  |  | Protéine native | Kinoïde |
|---|---|---|---|
| Souris 1 | J-2 | 0,15 | 0,2 |
|  | J40 | 1,7 | 1,9 |
| Souris 2 | J-2 | 0,1 | 0,1 |
|  | J40 | 1,9 | 1,6 |
| Souris 3 | J-2 | 0,25 | 0,15 |
|  | J40 | 1,9 | 2 |
| Souris 4 | J-2 | 0,1 | 0,1 |
|  | J40 | 0,15 | 0,1 |
| Souris 5 | J-2 | 0,2 | 0,3 |
|  | J40 | 0,3 | 0,2 |
| Souris 6 | J-2 | 0,2 | 0,15 |
|  | J40 | 0,1 | 0,1 |

Ces résultats montrent que les souris immunisées par le kinoïde produisent des anticorps capables de reconnaître de la même façon la protéine native et le kinoïde. D'autre part, cela confirme l'innocuité de l'immunisation par les kinoïdes car les souris ont très bien toléré l'immunisation.

Exemple 13. Traitement de l'IL10 par alkylation réductive.

3 ml d'IL10 à la concentration de 1 mg/ml en tampon borate 0,2 M de pH 9 ont été additionnés sous agitation de 1,5 mg de borohydrure de sodium en poudre. A cette solution a été ajoutée une solution de formaldéhyde à 37% en 5 portions successives de 15 ml sur une période de 30 mn Le mélange réactionnel a été dialysé à l'issue de cette période contre PBS.

Exemple 14. Perte de l'activité biologique de l'IL 10 inactivée par alkylation réductive.

L'IL10 a une action proliférative sur les cellules de la lignée mastocytaire de souris MC/9. On met en culture des cellules MC/9 en RPMI, 10% SVF, 2 mM L-glutamine, 0,05 mM 2-mercaptoéthanol, 100 U/ml de GM-CSF à la concentration initiale de $2\,10^5$/ml. Après 2 jours les cellules sont lavées et re-suspendues à $2\,10^5$/ml dans RPMI, 10 % SVF.
100 ml de cellules sont mises dans des puits de microplaques de culture à 96 puits. A ces puits, sont ajoutés 100 ml d'IL10 native ou inactivée selon l'exemple 13 à des dilutions différentes. Les cellules sont cultivées durant 40 heures, puis de la thymidine tritiée est ajoutée et après 8 heures, l'ADN cellulaire est capté sur filtre (MASH, Coulter) et la prolifération cellulaire est évaluée dans un compteur $\beta$.
Les résultats de prolifération (cpm) obtenus sont les suivants, en fonction de la quantité d'IL10 versée par puits :

| Quantité par puits : | IL10 natif | Kinoïde IL10 |
|---|---|---|
| 100 ng | 98 000 | 1600 |
| 10 ng | 88 000 | 900 |
| 1 ng | 36 000 | 1200 |
| 100 pg | 6 000 | 1000 |
| 10 pg | 1 000 | 800 |
| contrôle (pas d'IL10) | 1100 | 1100 |

Ces résultats montrent bien que le kinoïde IL10 a été effectivement inactivé par l'alkylation réductive.

Exemple 15. Immunogénicité de l'IL10 inactivée par alkylation réductive.

Des kinoïdes préparés selon l'exemple 13 ont été utilisés pour immuniser des souris Le protocole d'immunisation est celui classiquement utilisé : on injecte à des souris, (en im), 100 ml d'émulsion (1 : 1) en adjuvant complet de Freund contenant 20 mg de produit au jour 0, avec rappel en adjuvant incomplet de Freund de 5 mg aux jours 21 et 35. Le sérum des souris est prélevé aux jours -2 et 40 et analysé par ELISA sur des plaques sensibilisées avec la protéine native (non traitée chimiquement) et avec du kinoïde. Les sérums ont été testés à une dilution au 1/500.
Les résultats, exprimés en densité optique, obtenus sur 3 souris immunisées (1 à 3) et 3 souris non immunisées (4 à 6), sont rapportés dans le tableau suivant :

| | | Protéine native | Kinoïde |
|---|---|---|---|
| Souris 1 | J-2 | 0,2 | 0,15 |
| | J40 | 2 | 2,1 |
| Souris 2 | J-2 | 0,15 | 0,3 |
| | J40 | 1,5 | 1,8 |
| Souris 3 | J-2 | 0,2 | 0,1 |
| | J40 | 1,9 | 1,7 |
| Souris 4 | J-2 | 0,1 | 0,1 |
| | J40 | 0,1 | 0,15 |
| Souris 5 | J-2 | 0,15 | 0,25 |
| | J40 | 0,1 | 0,1 |
| Souris 6 | J-2 | 0,2 | 0,2 |
| | J40 | 0,1 | 0,25 |

Ces résultats montrent que les souris immunisées par le kinoïde produisent des anticorps capables de reconnaître de la même façon la protéine native et le kinoïde. D'autre part, cela confirme l'innocuité de l'immunisation par les kinoïdes car les souris ont très bien toléré l'immunisation.

Exemple 16. Inactivation de l'IFNγ par un anhydride.

L'IFNγ en tampon phosphate 0,1 M, pH 8,1 à la concentration de 1 mg par ml, refroidi dans un bain de glace a été additionné d'une solution d'anhydride maléique 1 M dans du dioxane redistillé. L'addition est effectuée, sous agitation, par petites fractions à intervalles de 5 mn, tout en maintenant le pH à 8,0 par addition de NaOH 0,05 N pendant tout le temps de réaction. La réaction a été arrêtée après avoir poursuivi l'agitation pendant encore 30 mn par dialyse contre PBS.

Exemple 17. Immunogénicité de l'IFNγ inactivée par un anhydride.

Des kinoïdes préparés selon l'exemple 16 ont été utilisés pour immuniser des souris Le protocole d'immunisation est celui classiquement utilisé : on injecte à des souris, (en im), 100 ml d'émulsion (1 : 1) en adjuvant complet de Freund contenant 20 mg de produit au jour 0, avec rappel en adjuvant incomplet de Freund de 5 mg aux jours 21 et 35. Le sérum des souris est prélevé aux jours -2 et 40 et analysé par ELISA sur des plaques sensibilisées avec la protéine native (non traitée chimiquement) et avec du kinoïde. Les sérums ont été testés à une dilution au 1/500.

Les résultats, exprimés en densité optique, obtenus sur 3 souris immunisées (1 à 3) et 3 souris non immunisées (4 à 6), sont rapportés dans le tableau suivant :

|  |  | Protéine native | Kinoïde |
|---|---|---|---|
| Souris 1 | J-2 | 0,3 | 0,2 |
|  | J40 | 2,2 | 1,9 |
| Souris 2 | J-2 | 0,2 | 0,1 |
|  | J40 | 1,9 | 1,7 |
| Souris 3 | J-2 | 0,15 | 0,25 |
|  | J40 | 1,9 | 2,1 |
| Souris 4 | J-2 | 0,15 | 0,1 |
|  | J40 | 0,1 | 0,25 |
| Souris 5 | J-2 | 0,25 | 0,1 |
|  | J40 | 0,15 | 0,15 |
| Souris 6 | J-2 | 0,1 | 0,2 |
|  | J40 | 0,15 | 0,1 |

Ces résultats montrent que les souris immunisées par le kinoïde produisent des anticorps capables de reconnaître de la même façon la protéine native et le kinoïde. D'autre part, cela confirme l'innocuité de l'immunisation par les kinoïdes car les souris ont très bien toléré l'immunisation.

Exemple 18. Traitement de l'IL13 par p-hydroxyphénylglyoxal.

Une solution d'IL13 de à 1 mg/ml dans du PBS a été additionnée, sous agitation, de 100 ml d'une solution d'hydroxyphénylglyoxal à 20 mg/ml dans le même tampon par ml de solution d'IFNγ. La réaction a été poursuivie pendant 1 heure à la température de 37°C, et le mélange réactionnel a été dialysé contre PBS.

Exemple 19. Perte de l'activité biologique de l'IL13 traité par p-hydroxyphénylglyoxal.

L'IL13 a une activité proliférative sur la lignée humaine érythroleucémique TF-1. Comme dans les expériences précédentes, les cellules TF-1 sont mises en culture en RPMI, 5% SVF, puis distribuées sur microplaques à $10^4$ cellules par puits dans 100 ml. A ces puits sont ajoutées des dilutions croissantes d'IL13 native ou inactivée selon l'exemple 18.

Après 40 heures, ajouter la thymidine tritiée. Après 8 heures, on recueille l'ADN des cellules sur filtre (MASH, Coulter) et ensuite on mesure la radioactivité au compteur β.

Les résultats de prolifération (en cpm) obtenus avec l'IL13 sont les suivants :

| Quantité ajoutée par puits | IL13 native | kinoïde IL13 |
|---|---|---|
| 100 ng | 76 000 | 2000 |
| 10 ng | 80 000 | 1 200 |
| 1 ng | 62 000 | 900 |
| 100 pg | 21 000 | 700 |
| 10 pg | 3 000 | 1 100 |
| contrôle | 900 | 900 |

On voit que l'inactivation a été efficace vu que l'IL13 native diluée $10^4$ fois (10 pg) est plus active que le kinoïde à la même concentration initiale non dilué (100 ng).

Exemple 20. Immunogénicité de L'IL13 inactivée par p-hydroxyphénylglioxal

Des kinoïdes préparés selon l'exemple 18 ont été utilisés pour immuniser des souris. Le protocole d'immunisation est celui classiquement utilisé : on injecte à des souris, (en im), 100 ml d'émulsion (1 : 1) en adjuvant complet de Freund contenant 20 mg de produit au jour 0, avec rappel en adjuvant incomplet de Freund de 5 mg aux jours 21 et 35. Le sérum des souris est prélevé aux jours -2 et 40 et analysé par ELISA sur des plaques sensibilisées avec la protéine native (non traitée chimiquement) et avec du kinoïde. Les sérums ont été testés à une dilution au 1/500.

Les résultats, exprimés en densité optique, obtenus sur 3 souris immunisées (1 à 3) et 3 souris non immunisées (4 à 6), sont rapportés dans le tableau suivant :

| | | Protéine native | Kinoïde |
|---|---|---|---|
| Souris 1 | J-2 | 0,3 | 0,25 |
| | J40 | 1,8 | 1,8 |
| Souris 2 | J-2 | 0,2 | 0,15 |
| | J40 | 2,1 | 2 |
| Souris 3 | J-2 | 0,35 | 0,15 |
| | J40 | 2 | 1,8 |
| Souris 4 | J-2 | 0,3 | 0,1 |
| | J40 | 0,1 | 0,1 |
| Souris 5 | J-2 | 0,1 | 0,25 |
| | J40 | 0,2 | 0,15 |
| Souris 6 | J-2 | 0,2 | 0,1 |
| | J40 | 0,3 | 0,15 |

Ces résultats montrent que les souris immunisées par le kinoïde produisent des anticorps capables de reconnaître de la même façon la protéine native et le kinoïde. D'autre part, cela confirme l'innocuité de l'immunisation par les kinoïdes car les souris ont très bien toléré l'immunisation.

Exemple 21. Inactivation de l'IL4 par alkylation réductive.

A une solution d'IL4 à la concentration de 1 mg/ml (5 ml) en tampon borate 0,2 M de pH 9, a été ajouté sous agitation, 1 mg de borohydrure de sodium et aussitôt après 50 ml de solution de formaldéhyde à 37% en 5 fractions successives à intervalles de 5 mn Après la dernière addition de l'aldéhyde, le mélange a été agité pendant encore 15 mn avant d'être dialysé contre PBS.

On a réalisé le même test que celui décrit dans l'exemple 8 pour mesurer l'inactivation du kinoïde IL4 par alkylation réductive. Le résultat obtenu a été similaire à celui de l'exemple 8 car les cellules stimulées par IL4 natif proliféraient et pas les cellules stimulées par le kinoïde.

Exemple 22. Immunogénicité de l'IL4 inactivée par alkylation réductive.

Des kinoïdes préparés selon l'exemple 21 ont été utilisés pour immuniser des souris. Le protocole d'immunisation est celui classiquement utilisé : on injecte à des souris, (en im), 100 ml d'émulsion (1 : 1) en adjuvant complet de Freund contenant 20 mg de produit au jour 0, avec rappel en adjuvant incomplet de Freund de 5 mg aux jours 21 et 35. Le sérum des souris est prélevé aux jours -2 et 40 et analysé par ELISA sur des plaques sensibilisées avec la protéine native (non traitée chimiquement) et avec du kinoïde. Les sérums ont été testés à une dilution au 1/500.

Les résultats, exprimés en densité optique, obtenus sur 3 souris immunisées (1 à 3) et 3 souris non immunisées (4 à 6), sont rapportés dans le tableau suivant :

|  |  | Protéine native | Kinoïde |
|---|---|---|---|
| Souris 1 | J-2 | 0,25 | 0,2 |
|  | J40 | 1,7 | 2 |
| Souris 2 | J-2 | 0,1 | 0,15 |
|  | J40 | 1,8 | 1,8 |
| Souris 3 | J-2 | 0,1 | 0,2 |
|  | J40 | 2 | 1,9 |
| Souris 4 | J-2 | 0,25 | 0,1 |
|  | J40 | 0,15 | 0,2 |
| Souris 5 | J-2 | 0,2 | 0,2 |
|  | J40 | 0,3 | 0,1 |
| Souris 6 | J-2 | 0,1 | 0,1 |
|  | J40 | 0,15 | 0,1 |

Ces résultats montrent que les souris immunisées par le kinoïde produisent des anticorps capables de reconnaître de la même façon la protéine native et le kinoïde. D'autre part cela confirme l'innocuité de l'immunisation par les kinoïdes car les souris ont très bien toléré l'immunisation.

Exemple 23. Inactivation du TNFα par l'éthylacétimidate.

A 2 ml d'une solution d'IFNα à 1 mg/ml en tampon borate 0,1 M, pH 8,5 refroidie dans un bain de glace ont été ajoutés, sous agitation 50 ml d'une solution d'éthylacétimidate, HCl à 12,5 mg/ml dans NaOH 5 N. La réaction a été poursuivie pendant 1 h, à 0°C, sous agitation, tout en maintenant le pH à 8,5 par addition de NaOH diluée. La réaction a été stoppée par dialyse contre PBS.

L'inactivation du TNFα obtenu a été évaluée selon le même test que celui décrit à l'exemple 5. Les résultats ont été similaires et il n'y avait plus de lyse des cellules en présence du kinoïde.

Exemple 24. Immunogénicité du TNFα inactivé par l'éthylacétimidate.

Des kinoïdes préparés selon l'exemple 23 ont été utilisés pour immuniser des souris Le protocole d'immunisation est celui classiquement utilisé : on injecte à des souris (en im) 100 ml d'émulsion (1 : 1) en adjuvant complet de Freund contenant 20 mg de produit au jour 0, avec rappel en adjuvant incomplet de Freund de 5 mg aux jours 21 et 35. Le sérum des souris est prélevé aux jours -2 et 40 et analysé par ELISA sur des plaques sensibilisées avec la protéine native (non traitée chimiquement) et avec du kinoïde. Les sérums ont été testés à une dilution au 1/500.

Les résultats, exprimés en densité optique, obtenus sur 3 souris immunisées (1 à 3) et 3 souris non immunisées (4 à 6), sont rapportés dans le tableau suivant :

|  |  | Protéine native | Kinoïde |
|---|---|---|---|
| Souris 1 | J-2 | 0,1 | 0,2 |
|  | J40 | 2,1 | 2 |
| Souris 2 | J-2 | 0,1 | 0,2 |
|  | J40 | 2 | 2,2 |
| Souris 3 | J-2 | 0,2 | 0,15 |
|  | J40 | 2,1 | 1,9 |
| Souris 4 | J-2 | 0,25 | 0,1 |
|  | J40 | 0,2 | 0,2 |
| Souris 5 | J-2 | 0,2 | 0,15 |
|  | J40 | 0,15 | 0,2 |
| Souris 6 | J-2 | 0,3 | 0,1 |
|  | J40 | 0,1 | 0,25 |

Ces résultats montrent que les souris immunisées par le kinoïde produisent des anticorps capables de reconnaître de la même façon la protéine native et le kinoïde. D'autre part cela confirme l'innocuité de l'immunisation par les kinoïdes car les souris ont très bien toléré l'immunisation.

Exemple 25. Traitement de l'IL1 par carboxyméthylation.

2 mg d'IL1 ont été dissous dans 2 ml de tampon Tris 0,3 M, pH 8 contenant de la guanidine 6 M et du dithiothreïtol (DTT) 10 mM précédemment désaérés par purge avec de l'azote. A cette solution ont été ajoutés 40 ml d'une solution d'acide iodacétique 0,5 M désaérée et le mélange réactionnel a été incubé pendant 90 mn à 37°C sous une barrière d'azote. La réaction a été alors bloquée par addition de 10 ml d'une solution 1 : 10 de β-mercaptoéthanol. L'incubation a été prolongée pendant encore 1 heure et le mélange réactionnel a été dialysé successivement contre de l'urée 8 M, 4 M, 2 M et finalement contre PBS.

Exemple 26. Perte de l'activité biologique de l'IL1 traitée par carboxyméthylation.

L'activité biologique de l'IL1 (IL1α ou IL1β) peut être mise en évidence en utilisant les cellules de la lignée de souris NOB qui sont induites à produire de l'IL2 en présence d'IL1. Les cellules sont cultivées en RPMI, 5 % SVF. En phase de croissance, elles sont lavées (centrifugation-lavage) et re-suspendues à la concentration de $10^5$ cellules/ml. Les cellules sont alors aliquotées sur microplaques à 100 ml par puits, et on leur ajoute 100 ml d'IL1 native ou traitée selon l'exemple 25 à dilutions croissantes. Les plaques sont alors incubées à 37°C pendant 36 heures et la production d'IL2 dans le surnageant est mesurée par ELISA (Quantikine, R&D Diagnostics).
Les résultats obtenus (en densité optique) pour l'IL1 et le kinoïde de l'exemple 25 sont les suivants :

| Quantité par puits : | IL1 natif | kinoïde |
|---|---|---|
| 1 mg | 2,1 | 0,4 |
| 100 pg | 2,2 | 0,2 |
| 10 pg | 1,3 | 0,3 |
| 1 pg | 0,6 | 0,2 |
| 0,1 pg | 0,3 | 0,2 |
| contrôle | 0,2 | 0,2 |

On voit donc que le kinoïde a perdu son activité par rapport à l'IL1 native de plus d'un facteur 1000.

Exemple 27. Immunogénicité de l'IL1 carboxyméthylée.

Des kinoïdes préparés selon l'exemple 25 ont été utilisés pour immuniser des souris Le protocole d'immunisation est celui classiquement utilisé : on injecte à des souris (en im), 100 ml d'émulsion (1 : 1) en adjuvant complet de Freund contenant 20 mg de produit au jour 0, avec rappel en adjuvant incomplet de Freund de 5 mg aux jours 21 et 35. Le sérum des souris est prélevé aux jours -2 et 40 et analysé par ELISA sur des plaques sensibilisées avec la protéine native (non traitée chimiquement) et avec du kinoïde. Les sérums ont été testés à une dilution au 1/500.
Les résultats, exprimés en densité optique, obtenus sur 3 souris immunisées (1 à 3) et 3 souris non immunisées (4 à 6), sont rapportés dans le tableau suivant :

|  |  | Protéine native | Kinoïde |
|---|---|---|---|
| Souris 1 | J-2 | 0,3 | 0,2 |
|  | J40 | 1,6 | 1,7 |
| Souris 2 | J-2 | 0,2 | 0,3 |
|  | J40 | 1,8 | 2 |
| Souris 3 | J-2 | 0,1 | 0,2 |
|  | J40 | 1,9 | 1,6 |
| Souris 4 | J-2 | 0,15 | 0,2 |
|  | J40 | 0,1 | 0,25 |
| Souris 5 | J-2 | 0,1 | 0,2 |
|  | J40 | 0,3 | 0,3 |
| Souris 6 | J-2 | 0,25 | 0,15 |
|  | J40 | 0,3 | 0,1 |

Ces résultats montrent que les souris immunisées par le kinoïde produisent des anticorps capables de reconnaître de la même façon la protéine native et le kinoïde. D'autre part, cela confirme l'innocuité de l'immunisation par les kinoïdes car les souris ont très bien toléré l'immunisation.

Exemple 28. Inactivation de l'IFN$\alpha$ par maléimidation.

A 1,7 ml d'une solution d'IFN$\alpha$ à 1 mg/ml dans du tampon Tris 30 mM, EDTA 1 mM, de pH 7,8 ont été ajoutés sous agitation 50 ml d'une solution d'acide $\beta$-maléimidopropionique à 2 mg/ml dans le même tampon. Après réaction, pendant 15 mn à la température du laboratoire, le mélange réactionnel a été dialysé contre PBS.
Le degré d'inactivation de l'IFN$\alpha$ maléimidé a été évalué par le test d'inhibition de la lyse des cellules MDBK par le virus VSV (même test que pour l'IFN$\gamma$ en exemple 2). L'activité biologique du kinoïde était inhibée à plus de 99,9 % comparée à l'IFN$\alpha$ natif.

Exemple 29. Immunogénicité de l'IFN$\alpha$ maléimidée.

Des kinoïdes préparés selon l'exemple 28 ont été utilisés pour immuniser des souris. Le protocole d'immunisation est celui classiquement utilisé : on injecte à des souris (en im), 100 ml d'émulsion (1 : 1) en adjuvant complet de Freund contenant 20 mg de produit au jour 0, avec rappel en adjuvant incomplet de Freund de 5 mg aux jours 21 et 35. Le sérum des souris est prélevé aux jours -2 et 40 et analysé par ELISA sur des plaques sensibilisées avec la protéine native (non traitée chimiquement) et avec du kinoïde. Les sérums ont été testés à une dilution au 1/500.
Les résultats, exprimés en densité optique, obtenus sur 3 souris immunisées (1 à 3) et 3 souris non immunisées (4 à 6), sont rapportés dans le tableau suivant :

|  |  | Protéine native | Kinoïde |
|---|---|---|---|
| Souris 1 | J-2 | 0,1 | 0,25 |
|  | J40 | 1,7 | 2 |

(suite)

|  |  | Protéine native | Kinoïde |
|---|---|---|---|
| Souris 2 | J-2 | 0,15 | 015 |
|  | J40 | 1,9 | 2,1 |
| Souris 3 | J-2 | 0,35 | 0,3 |
|  | J40 | 1,8 | 1,9 |
| Souris 4 | J-2 | 0,15 | 0,1 |
|  | J40 | 0,25 | 0,3 |
| Souris 5 | J-2 | 0,1 | 0,3 |
|  | J40 | 0,2 | 0,15 |
| Souris 6 | J-2 | 0,25 | 0,1 |
|  | J40 | 0,2 | 0,1 |

Ces résultats montrent que les souris immunisées par le kinoïde produisent des anticorps capables de reconnaître de la même façon la protéine native et le kinoïde. D'autre part, cela confirme l'innocuité de l'immunisation par les kinoïdes car les souris ont très bien toléré l'immunisation.

## Revendications

1. Un dérivé de cytokine ou d'un fragment de cytokine de formule

$$Cy\text{-}(X\text{-}R)_n$$

dans laquelle Cy représente une cytokine ou un fragment de cytokine, - (X-R) représente une fonction libre d'un acide aminé constituant la cytokine ou le fragment de cytokine où X représente un groupement NH ou un atome de soufre, n représente un entier allant de 1 à 70, et R représente un groupement chimique conservant audit dérivé de cytokine ou audit fragment des propriétés immunogènes suffisantes pour créer des anticorps neutralisant ou bloquant ladite cytokine native et en même temps faisant perdre au moins 90% des propriétés biologiques toxiques de ladite cytokine native, **caractérisé en ce que** :

(i) R est un groupement carboxamide, N-alkylmaléimide, thionitrobenzoïque ou un radical formé par réaction d'une fonction thiol avec le 4-chloro-7-sulfobenzofurazane d'ammonium, le N-[iodoéthyl]-trifluoroacétamide ou le N-(6-[7-amino-4-méthylcoumarin-3-acétamido]hexyl)-3'-(2'-pyridyldithio)propionamide, ou
(ii) R est un radical formé par réaction d'une fonction amino avec l'éthylacétimidate, un anhydride, le 2-iminothiolane-HCl, le N-succinimidyl S-acétylthioacétate, le sulfosuccinimidyl-acétate, le sulfosuccinimidyl-4-0-[4,4'-diméthoxytrityl]butyrate, le succinimidyl 7-amino-4-méthylcoumarin-3-acétate, le sulfosuccinimidyl 7-amino-4-méthylcoumarin-3-acétate ou le phénylglyoxal.

2. Un dérivé de cytokine ou un fragment selon la revendication 1 **caractérisé en ce que** ladite cytokine ou ledit fragment est ou est issu de IFNγ, TNFα, IL1, IL4, IL6, IL10, IL13, des interleukines -IL1, IL2, IL3, des Transforming Growth Factors α et β, des interférons α, β, τ(tau), des Tumor Necrosis Factors -α et β, des chimiokines.

3. Procédé de préparation d'une cytokine ou d'un fragment tel que défini dans l'une des revendications 1 ou 2, **caractérisé en ce que** l'on soumet ladite cytokine ou ledit fragment à l'action d'un réactif capable de greffer un groupement R sur ladite cytokine ou ledit fragment, pour obtenir le composé attendu que l'on isole.

4. Un dérivé de cytokine ou fragment selon la revendication 1 ou 2, pour son utilisation dans une méthode de traitement thérapeutique du corps humain ou animal.

5. Une composition pharmaceutique **caractérisée en ce qu'**elle renferme à titre de principe actif l'un au moins des cytokines ou fragments tels que définis à la revendication 1 ou 2 ainsi qu'un excipient pharmaceutiquement acceptable.

6. Un vaccin **caractérisé en ce qu'**il renferme à titre de principe actif l'un au moins des cytokines ou fragments tels que définis à l'une des revendications 1 ou 2.

7. Utilisation d'une cytokine ou fragment tel que défini à l'une des revendications 1 ou 2, pour l'obtention d'un médicament destiné à lutter contre la surproduction de ladite cytokine native.

**Claims**

1. A derivative of cytokine or a fragment of cytokine of formula :

$$Cy\text{-}(X\text{-}R)_n$$

in which Cy represents a cytokine or a cytokine fragment, -(X-R) represents a free function of an amino acid constituting the cytokine or the cytokine fragment where X represents an NH group or a sulphur atom, n represents an integer from 1 to 70, and R represents a chemical group preserving for the said cytokine or said fragment sufficient immunogenic properties to create antibodies neutralising or blocking the said native cytokine and at the same time bringing about the loss of at least 90% of the toxic biological properties of the said native cytokine, **characterised in that**

(i) R is a carboxamide, N-alkylmaleimide, thionitrobenzoic group or a radical formed by reaction of a thiol function with ammonium 4-chloro-7-sulphobenzofurazane, N-[iodoethy]-trifluoroacetamide or N-(6-[7-amino-4-methylcoumarin-3-acetamido]hexyl)-3'-(2'-pyridyldithio) propionamide, or
(ii) R is a radical formed by reaction of an amino function with ethylacetimidate, an anhydride, 2-iminothiolane-HCl, N-succinimidyl S-acetylthioacetate, sulphosuccinimidyl-acetate, sulphosuccinimidyl-4-O-[4-4'-dimethoxytrityl]butyrate, succinimidyl 7-amino-4-methylcoumarin-3-acetate, sulphosuccinimidyl 7-amino-4-methylcoumarin-3-acetate or phenylglyoxal.

2. A cytokine derivative or a fragment according to claim 1 **characterised in that** the said cytokine or said fragment is or has come from IFNγ, TNFα, IL1, IL4, IL6, IL10, IL13, interleukines -IL1, IL2, IL3, Transforming Growth Factors α and β, interferons α, β, γ, τ (tau), Tumour Necrosis Factors-α and β, and chemiokines.

3. Method for producing cytokine or a fragment as defined in any one of claims 1 and 2, **characterised in that** the said cytokine or said fragment is subjected to the action of a reagent capable of grafting a R group onto the said cytokine or said fragment, in order to obtain the expected compound which is isolated.

4. A cytokine derivative or fragment according to claim 1 or claim 2, for its use in a method of therapeutic treatment of the human or animal body.

5. A pharmaceutical composition **characterised in that** it contains as active ingredient at least one of the cytokines or fragments as defined in claim 1 or claim 2 as well as a pharmaceutically acceptable excipient.

6. A vaccine **characterised in that** it contains as active ingredient at least one of the cytokines or fragments as defined in any one of claims 1 and 2.

7. Use of a cytokine or fragment such as defined any one of claims 1 and 2, in order to obtain a chug intended to combat overproduction of the said native cytokine.

**Patentansprüche**

1. Derivat eines Cytokins oder eines Cytokinfragments der Formel

$$Cy\text{-}(X\text{-}R)_n,$$

wobei Cy für ein Cytokin oder Cytokinfragment steht, -(X-R) für eine freie Funktion einer Aminosäure steht, die eine Komponente des Cytokins oder Cytokinfragments ist, wobei X für eine NH-Gruppe oder ein Schwefelatom steht, n für eine ganze Zahl von 1 bis 70 steht und R für eine chemische Gruppe steht, die dem Cytokinderivat oder Fragment ausreichende immunogene Eigenschaften bewahrt, um Antikörper zu erzeugen, die das native Cytokin neutralisieren oder blockieren, und gleichzeitig wenigstens 90% der toxischen biologischen Eigenschaften des nativen Cytokins verlieren lässt;
**dadurch gekennzeichnet, dass**:

(i) R eine Carboxamid-, N-Alkylmaleinimid-, Thionitrobenzoesäuregruppe oder ein durch Reaktion einer Thiolfunktion mit Ammonium-4-chlor-7-sulfobenzofurazan, N-[Iodethyl]trifluoracetamid oder N-(6-[7-Amino-4-methylcumarin-3-acetamido]hexyl)-3'-(2'-pyridyldithio)propionamid gebildeter Rest ist; oder

(ii) R ein Rest ist, der durch Reaktion einer Aminofunktion mit Ethylacetimidat, einem Anhydrid, 2-Iminothiolan-HCl, N-Succinimidyl-S-acetylthioacetat, Sulfosuccinimidylacetat, Sulfosuccinimidyl-4-O-[4,4'-dimethoxytrityl]butyrat, Succinimidyl-7-amino-4-methylcumarin-3-acetat, Sulfosuccinimidyl-7-amino-4-methylcumarin-3-acetat oder Phenylglyoxal gebildet ist.

2.  Derivat eines Cytokins oder eines Fragments gemäß Anspruch 1, **dadurch gekennzeichnet, dass** es sich bei dem Cytokin um IFN$\gamma$, TNF$\alpha$, IL-1, IL-4, IL-6, IL-10, IL-13, die Interleukine -IL-1, IL-2, IL3, die Transforming-Wachstumsfaktoren $\alpha$ und $\beta$, die Interferone $\alpha$, $\beta$, $\tau$ (tau), die Tumornekrosefaktoren $\alpha$ und $\beta$ oder Chemokine handelt bzw. das Fragment aus diesen hervorgegangen ist.

3.  Verfahren zur Herstellung eines Cytokins oder Fragments, wie es in einem der Ansprüche 1 oder 2 definiert ist, **dadurch gekennzeichnet, dass** das Cytokin oder Fragment der Einwirkung eines Reagens ausgesetzt wird, das eine Gruppe R mit dem Cytokin oder Fragment verknüpfen kann, so dass man die erwartete Verbindung erhält, die dann isoliert wird.

4.  Derivat eines Cytokins oder eines Fragments gemäß Anspruch 1 oder 2 zur Verwendung in einem Verfahren zur therapeutischen Behandlung des menschlichen oder tierischen Körpers.

5.  Pharmazeutische Zusammensetzung, **dadurch gekennzeichnet, dass** sie als Wirkstoff wenigstens eines der Cytokine oder Fragmente, wie sie in Anspruch 1 oder 2 definiert sind, sowie einen pharmazeutisch annehmbaren Arzneimittelhilfsstoff umfasst.

6.  Impfstoff, **dadurch gekennzeichnet, dass** er als Wirkstoff wenigstens eines der Cytokine oder Fragmente, wie sie in einem der Ansprüche 1 oder 2 definiert sind, umfasst.

7.  Verwendung eines Cytokins oder Fragments, wie sie in einem der Ansprüche 1 oder 2 definiert sind, zur Gewinnung eines Medikaments zur Bekämpfung einer Überproduktion des nativen Cytokins.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 9200544 W
- WO 8606414 A
- EP 0220273 A
- US 8600831 W

**Littérature non-brevet citée dans la description**

- **A. GRINGERI et al.** *J of AIDS,* 1999, vol. 20, 358-370
- **LANDEGREN et al.** *J Immunol Méthods,* 1984, vol. 67, 379